# FASCICULE DE BREVET EUROPEEN

(11) **EP 2 046 407 B1**
(45) Date de publication et mention de la délivrance du brevet: **18.03.2015**
(21) Numéro de dépôt: 07730855.9
(22) Date de dépôt: 24.01.2007
(51) Int. Cl.: A61L 27/22, A61K 38/42, A61P 17/02

(54) **PREPARATION IMPLANTABLE, UTILISABLE NOTAMMENT POUR LE COMPLEMENT TISSULAIRE ET LA CICATRISATION**
IMPLANTIERBARE ZUBEREITUNG, INSBESONDERE FÜR GEWEBEKOMPLEMENT UND VERNARBUNG
IMPLANTABLE PREPARATION, USEFUL MORE PARTICULARLY FOR TISSUE COMPLEMENT AND CICATRISATION

(30) Priorité: 02.08.2006 WO PCT/FR2006/001880
(43) Date de publication de la demande: 15.04.2009
(73) Titulaire: Khorionyx, 69890 La Tour de Salvagny (FR)
(72) Inventeur: TAYOT, Jean-Louis, 69890 La Tour De Salvagny (FR)
(74) Mandataire: Colombet, Alain André
(86) Numéro de dépôt international: PCT/FR2007/000137
(87) Numéro de publication internationale: WO 2008/015319

(56) Documents cités:
- EP-A- 1 080 737
- FR-A- 2 854 801
- US-A- 2 460 550
- US-A- 2 597 432
- US-A1- 2004 248 774
- US-A1- 2005 136 122
- US-B1- 6 440 427

## Description

La présente invention a pour but de fournir de nouvelles préparations implantables ou injectables contenant de la globine, associée à des dispositifs ou des produits médicaux ne contenant pas de globine, dans toutes proportions. La globine peut par exemple représenter un constituant quantitativement minoritaire du produit final. Ces préparations peuvent se présenter, notamment, sous forme de poudres, compresses, pâtes, gels, suspensions, ou solutions injectables, ou de matériaux solides implantables, destinés notamment à combler ou réparer des défauts tissulaires, cutanés, conjonctifs, vasculaires, viscéraux ou osseux, et de manière générale à protéger ou combler des plaies et participer à leur cicatrisation. Différents biomatériaux constitués de polymères synthétiques ou naturels, ne contenant pas de globine, sont actuellement commercialisés et disponibles pour de telles applications médicales et chirurgicales. Cette invention a pour but principal d'améliorer leur biocompatibilité, en favorisant leur colonisation cellulaire, une intégration tissulaire harmonieuse et leur résorption.

Récemment de nouveaux matériaux et de nouvelles applications médicales ont été décrits à partir de globine insoluble à pH neutre dans des conditions physiologiques et ont fait l'objet d'une famille de brevets issus du brevet FR 03 05700 publié sous le numéro FR-A-2 854 801. Ces matériaux comprennent de la globine insoluble à pH neutre comme composant actif principal.

La globine est la protéine constitutive de l'hémoglobine qui, elle-même, contient 4 chaînes peptidiques (2 chaînes α et 2 chaînes P) chacune associée à un hème. L'hème est constitué d'une structure tétrapyrole contenant 1 atome de fer chargé positivement. Il y a 4 hèmes par molécule, responsables de la coloration rouge de l'hémoglobine.

Les procédés de préparation de la globine sont connus depuis très longtemps et ont été développés dans le but d'applications alimentaires ou pour la préparation de solutions pharmaceutiques injectables.

Contrairement à l'hémoglobine qui est très soluble à pH physiologique, la globine est remarquablement insoluble dans les mêmes conditions.

Un produit injectable associant une préparation soluble de globine acide avec de l'insuline a été mis au point, breveté et commercialisé : REINER (1939); REINER et al. (1939). Il permet après injection, une délivrance progressive de l'insuline à partir de ce complexe : RABINOWITCH et al. (1947) ; BERG et al. (1953). La globine qui compose cette préparation n'est pas chimiquement modifiée ; elle est insoluble à pH physiologique, présente à faible concentration, et elle n'est pas l'élément actif, ni l'élément principal de ce produit.

Il nous a paru intéressant de pouvoir fabriquer des biomatériaux composites préparés en intégrant de la globine insoluble à l'intérieur de matériaux polymériques déjà utilisés comme dispositifs médicaux, ou susceptibles de l'être. Même en proportion quantitative minoritaire, la présence de globine peut améliorer les propriétés biologiques de ces dispositifs médicaux et en particulier leur biocompatibilité, leur intégration cellulaire et tissulaire et leur résorption.

La présente invention se propose de fournir de nouveaux matériaux et préparations implantables dans l'organisme, comprenant de la globine naturelle ou modifiée et insoluble à pH physiologique. Ces implants ne présentent pas les inconvénients ou limitations des matériaux actuels, ne contenant pas de globine.

L'invention concerne notamment une préparation stérile implantable sur ou dans des tissus organiques, comprenant, sous forme de pâte, gel ou suspension injectable:
- un matériau de globine naturelle ou modifiée et insoluble au pH physiologique,
   biocompatibles et biodégradables dans l'organisme, et

- un agent polymérique de comblement ou augmentation tissulaire, à base d'acide hyaluronique réticulé.
- dans laquelle la proportion dudit matériau, par rapport à l'ensemble matériau plus agent polymérique est inférieure ou égale à 50%.

Par globine modifiée insoluble, on entend de la globine naturellement insoluble au pH physiologique, modifiée par adjonction d'une molécule ayant un caractère hydrophobe, par exemple, par estérification par un alcool gras.

Il est décrit unagent polymérique adhésif qui est un agent à base de l'un au moins des matériaux suivants : le fibrinogène, l'albumine, un dérivé adhésif et biocompatible du polyéthylène glycol, ou un polymère acrylique ou cyano-acrylique adhésif et biocompatible.

Par acide hyaluronique on entend notamment le mucopolysaccharide extrait de bactéries ou de tissus humains (tels que le cordon placentaire) ou animaux (tels que la crête de coq).

Par acide hyaluronique réticulé on entend notamment les produits injectables pour augmentation tissulaire commercialisés tels que par exemple Restylane®, Hylaform®, Juvederm®, Puragen Plus®. A titre d'exemple, la réticulation des chaines d'acide hyaluronique peut être réalisée par divers agents activateurs des réactions d'estérification, tels que les carbodiimides comme ceux décrits dans le brevet US 6,943,154, qui permettent la réaction des fonctions carboxyliques sur les fonctions alcools primaires de l'acide hyaluronique. D'autres agents bien connus pour réticuler les polysaccharides sont les composés di-époxy, tels que le 1-4 butanediol-diglycidylether qui réagissent à pH alcalin sur les fonctions alcool des polysaccharides. L'épichlorhydrine est aussi un agent bien connu pour réticuler les polysaccharides à pH alcalin. De tels exemples sont cités notamment dans le brevet US 5,234,991.

Par acide polylactique, on entend tout polymère constitué principalement de monomères tels que l'acide lactique, pouvant être polymérisés en présence d'autres monomères tels que l'acide glycolique, de manière à moduler leur temps de dégradation dans l'organisme, selon l'état de l'art.

Dans la préparation selon l'invention, la globine naturelle ou modifiée et insoluble au pH physiologique et/ou le matériau insoluble pouvant être obtenu à partir de globine modifiée, constituent, de préférence, même s'ils sont quantitativement minoritaires par rapport à l'agent polymérique associé, l'agent actif principal améliorant la biocompatibilité, la colonisation cellulaire ou l'intégration tissulaire de l'agent adhésif, ou de comblement ou d'augmentation, ou de cicatrisation, ce dernier ne présentant généralement que des qualités plus réduites, ou même inexistantes, dans ces domaines, et ayant essentiellement une fonction d'adhésion, de remplissage ou d'augmentation ou de protection. Lorsque, comme cela est généralement le cas, lesdits agents polymériques de comblement ou augmentation tissulaire, à base d'acide hyaluronique réticulé, présentent un fort pouvoir inflammatoire, la préparation selon l'invention permet de disposer, dans la masse injectée ou implantée, de zones peu ou pas inflammatoires, entraînant les avantages précités.

Selon un mode de mise en oeuvre préféré, la proportion de matériau de globine naturelle ou modifiée et insoluble au pH physiologique par rapport à l'ensemble en poids de ce matériau plus l'agent polymérique est inférieure à 50%, par exemple inférieure ou égale à 25% ou même inférieure ou égale à 10%, de façon à obtenir une préparation dans laquelle ledit matériau jouera son rôle d'amélioration de la biocompatibilité, de la colonisation cellulaire ou de l'intégration tissulaire, sans dilution substantielle de l'agent polymérique associé.

Il est décrit des préparations cicatrisantes, qui contiennent une proportion majoritaire de cellulose oxydée, celle-ci assure sa fonction usuelle de cicatrisation, le composant de globine ou dérivé assurant les fonctions mentionnées ci-dessus, mais pouvant, en plus, en fonction de la quantité présente, jouer un rôle cicatrisant propre, comme par exemple dans la demande de brevet FR A 2 854 801.

De même, dans les préparations de comblement, ce composant pourra, en outre, en fonction de la proportion présente, jouer un rôle propre de comblement, comme décrit dans la demande de brevet français précité.

Par pH physiologique on entend notamment un pH compris entre 6 et 8, préférablement entre 6,5 et 7,5 et préférentiellement la gamme des pH physiologiques. Par implantable, on entend la faculté d'être implanté ou injecté dans ou sur l'organisme, dans des tissus ou en contact avec eux, y compris sur la peau ou sur des plaies externes, l'administration intravasculaire étant exclue. Cela suppose la possibilité de les stériliser, ou de les préparer directement sous forme stérile.

La stérilisation peut être obtenue notamment par irradiation beta ou gamma de 5 à 30kGray. Cette irradiation peut s'effectuer à température ambiante, ou pour certains produits fragiles, sous forme congelée en présence de carboglace. L'autoclavage, notamment à 120°C est possible lorsque la globine est insoluble.

Dans un mode de mise en oeuvre, le matériau est constitué de, ou obtenu à partir de globine naturelle insoluble, non chimiquement modifiée. La notion de globine naturelle inclut, bien entendu, une globine équivalente, qui serait obtenue par synthèse ou recombinaison génétique. De telles préparations ont été décrites dans la demande de brevet FR 0305700 précitée.

Il est décrit un matériau obtenu à partir de globine modifiée pour être soluble à pH physiologique, cette solubilité pouvant être totale ou partielle. De telles préparations ont été décrites dans la demande de brevet français FR 05 08392 déposée le 5 août 2005 et dans la demande de brevet PCT FR2006/001880 déposée le 2 août 2006, non encore publiées, et dont les contenus sont ici incorporés par référence.

Il est également décrit un matériau pouvant être obtenu à partir de globine modifiée soluble et de globine insoluble à pH physiologique. De telles préparations ont été décrites dans la demande de brevet FR 05 08392 précitée.

Ces préparations implantables insolubles de globine sont notamment sous forme de pâte ou gel, de matériaux solides, par exemple de poudre, de granules, de compresses, ou de films. Elles peuvent être insolubles dans un liquide physiologique, ou rendues insolubles après réticulation chimique de la globine soluble. Elles peuvent être injectables. Par injectable, on entend la propriété de pouvoir être injecté pour réaliser l'implantation locale, à l'exclusion de toute injection intraveineuse ou intra-artérielle qui sont formellement contre-indiquées.

Dans une forme de réalisation, ledit matériau de globine est sensiblement insoluble à pH physiologique, en étant préparé à partir de globine naturelle ou modifiée et insoluble, ou obtenu par réticulation de globine soluble modifiée.

La préparation peut se présenter sous forme de suspension, pâte, solution, ou gel, de préférence suffisamment fluide pour être injectable.

La préparation peut comprendre une poudre de globine insoluble.

Il est également décrit des préparations de globine pouvant être mélangées ou ajoutées à un agent polymérique adhésif, synthétique ou naturel, le mélange final pouvant former une colle adhésive sur les tissus organiques. Ledit agent polymérique adhésif est, de préférence, le composant liquide d'un kit de préparation de la colle, contenant les monomères non polymérisés ou les polymères non réticulés. Il n'est pas conseillé de mélanger la globine avec le composant de réticulation ou l'initiateur de polymérisation, car celui-ci serait consommé par la globine et rendu inactif pour la prise en masse de la colle. Ledit agent polymérique adhésif peut être lui-même sous forme de poudre, l'ensemble pouvant être formulé sous forme de spray ou aérosol et former une colle en présence d'un milieu liquide, notamment liquide physiologique.

Les préparations selon l'invention peuvent se présenter sous une forme d'un seul tenant, de préférence prêtes à l'emploi. Cependant elles peuvent aussi se présenter sous forme de deux ou plusieurs composants destinés à être associés ou mélangés, par exemple à l'aide d'un kit, de préférence sous forme stérile.

L'intérêt de cette nouvelle famille de produits réside notamment dans le fait qu'ils sont combinés à un biomatériau protéique insoluble à volonté, préparé à partir d'une protéine définie, pure, homologue ou autologue, parfaitement biocompatible avec les tissus environnants dans lesquels ils sont injectés. On peut ainsi obtenir, au sein de la masse adhésive implantée, un réseau biorésorbable, accessible aux cellules, qui dilue et diminue le caractère inflammatoire éventuel de l'implant, facilite la régénération tissulaire dans l'implant, son intégration harmonieuse dans les tissus voisins et sa résorption.

Parmi les polymères adhésifs naturels, le fibrinogène est déjà utilisé pour des applications médicales et chirurgicales diverses. Il est alors possible par exemple d'associer de manière nouvelle les colles à base de fibrinogène plasmatique, dites colles de fibrine, telles que Tisseel^{®}, Tissucol^{®}, Quixil^{®}, Hemaseal^{®}, Beriplast^{®} et d'autres avec les matériaux de globine soluble ou insoluble adhésifs ou non adhésifs.

Ces colles à base de fibrinogène ont été décrites notamment par la société Immuno dans les années 80 : Brevets US 4,298,598 et 4,362,567, ou plus récemment par la société Omrix : brevet US 6,019,993. L'association avec de la globine est une opportunité nouvelle qui permet une utilisation rationnelle des protéines sanguines et qui peut contribuer à éviter leur gaspillage, diminuer les doses nécessaires, améliorer leurs performances et augmenter le nombre de leurs applications. Ces colles chirurgicales selon l'invention comportent donc une teneur en matériau à base de fibrinogène, de préférence inférieure à la teneur habituelle, et un matériau soluble ou non pouvant être obtenu à partir de globine modifiée soluble ou de globine naturelle ou modifiée et insoluble, conformément à l'invention.

Parmi les polymères adhésifs naturels, l'albumine est déjà utilisée pour des applications médicales et chirurgicales diverses. Il est alors possible par exemple d'associer de manière nouvelle ces colles à base d'albumine avec de la globine soluble ou insoluble. Comme exemple de colle à base d'albumine, nous pouvons citer la colle Bioglue®, issue des travaux de N.Kowanko (US Patent 5385606), distribuée par la société Cryolife. D'autres produits voisins ont été décrits dans les demandes de brevets suivants: H.Goldmann, J.Wegmann, PCT/EP02/11880; T.H.Barrows, T.W.Lewis, M.T.Truong, PCT/US95/07947.

Parmi les polymères adhésifs synthétiques, à titre d'exemple, des dérivés du polyéthylène glycol ou des polymères acryliques ou cyano-acryliques sont déjà utilisés pour des applications médicales et chirurgicales diverses. Il est alors possible par exemple d'associer de manière nouvelle le composant monomérique de ces colles synthétiques avec de la globine soluble ou insoluble, pour en améliorer la biocompatibilité et l'intégration tissulaire et faciliter leur résorption. Parmi les colles biologiques synthétiques commercialisées, et à titre d'exemples non limitatifs, nous pouvons citer des colles à base de dérivés réactifs de polyéthylène glycol, telles que Coseal® développée par la société Cohesion, sur la base des brevets US5874500, US6166130, US6323278, US6624245 ; ou encore Duraseal® développée par la société Confluent Surgical ; les produits issus du brevet US 5986043 ; les produits FocaISeal® développés par la société Focal à partir de différents brevets dont les brevets US 5410016 et 5573934. Parmi les colles à base de monomères acryliques, nous pouvons citer à titre d'exemples non limitatifs le produit Indermil®, développé par la société Loctite et commercialisé par la société Kendall; le produit Dermabond® développé par la société Closure Médical et commercialisé par la société Ethicon. De nombreuses autres colles sont issues d'autres polymères synthétiques ou naturels, dont par exemple les dérivés de la cellulose, et d'autres demandes de brevets ou brevets parmi lesquels : EP0488629, EP0310919, US3640741.

La globine humaine homologue est préférable à une globine animale hétérologue, ce qui permet d'éviter au mieux toute réaction immunologique du patient à traiter, pendant ou après l'implantation.

La globine est facile à purifier à partir de globules rouges ou de sang humains. Les globules rouges humains sont disponibles en quantité importante à partir des dons périmés restant en stock dans les centres de transfusion sanguine et pour lesquels tous les tests préalables sanitaires ont été réalisés au moment du prélèvement. Les préparations de globine implantable, ou d'autres biomatériaux insolubles à base de la même globine, représentent donc une voie nouvelle pour satisfaire des applications biomédicales qui se développent de plus en plus, tout en permettant de valoriser le sang non utilisé ou les dons de sang périmés et d'éviter ou de diminuer leur destruction. En contraste avec d'autres protéines, dont les collagènes, la globine présente l'originalité de conserver ses caractères malgré un traitement alcalin prolongé, ou/et malgré une stérilisation par irradiation. Ceci facilite son emploi en toute sécurité, grâce à la garantie d'une puissante inactivation des agents infectieux ou transmissibles, potentiellement présents dans tout produit d'origine biologique.

La mise en oeuvre de l'invention est possible aussi à partir d'un prélèvement d'échantillon de sang du patient à traiter, par exemple d'environ 20 à 200 ml, et sa transformation en globine autologue, avec les mêmes méthodes que pour de grands volumes, puis sa transformation en biomatériau implantable destiné à l'invention.

La réalisation de l'invention peut nécessiter d'abord de recueillir et purifier les globules rouges dans ces échantillons de sang, ou liquides sanguins, par des opérations simples et déjà connues, par exemple selon le procédé suivant.

Les globules rouges sont récupérés par centrifugation à basse vitesse. Le surnageant plasmatique est séparé et remplacé par un liquide salin physiologique contenant 9g/l de NaCl. Après plusieurs lavages (3 à 5), la suspension de globules rouges est ainsi débarrassée des protéines du plasma. Le culot de globules rouges purifiés est additionné de 1 ou 2 volumes d'eau distillée pour réaliser un choc osmotique qui entraîne la lyse des membranes des globules rouges et libère l'hémoglobine en solution concentrée et purifiée. Une étape de centrifugation à haute vitesse (10 à 20.000 t/mn) permet d'éliminer les débris membranaires et cellulaires dans le culot. Une étape finale de filtration du surnageant sur membrane de porosité de 0,2 micron permet de préparer une solution d'hémoglobine purifiée et stérile, dépourvue de particules et dérivés d'origine tissulaire, cellulaire ou membranaire.

Ainsi, pour l'étape d'hémolyse des globules rouges, ceux-ci peuvent être purifiés, pour partir d'une solution d'hémoglobine purifiée déjà séparée des constituants plasmatiques. Mais cette étape peut être supprimée, notamment grâce à la propriété spécifique d'insolubilité de la globine dans une solution aqueuse de pH neutre, qui la distingue des autres protéines plasmatiques et permet de la séparer.

Le clivage Hème-Globine à pH acide a été décrit en présence d'alcool par SCHULZ dès 1898. ANSON et MIRSKY en 1930, puis ROSSI-FANELLI et coll. en 1958 utilisent l'acétone en présence d'acide à 0°C. TEALE (1959) préfère l'utilisation de la méthyl-éthyl cétone à la place de l'acétone. AUTIO et coll. (1984) séparent la globine à pH acide grâce à l'absorption et la précipitation de l'hème avec la carboxymethylcellulose soluble. La globine ainsi préparée est soluble à pH acide ou alcalin mais devient insoluble dès que le pH de la solution aqueuse est neutralisé entre pH 6 et 8. Grâce à cette propriété spécifique, il est possible de réaliser une précipitation sélective de la globine en solution aqueuse à pH neutre, de préférence en présence d'une faible concentration saline, par exemple, voisine de 5g/l de chlorure de sodium, conditions dans lesquelles les autres protéines plasmatiques sont solubles, ne co-précipitent pas avec la globine et sont séparées dans le surnageant. Cette méthode est utile lorsque l'on souhaite éviter une séparation préalable des globules rouges et du plasma, ce qui permet de congeler le sang aussitôt son prélèvement.

Dans ce cas, le sang est hémolysé, de préférence par congélation, puis décongélation, après quoi l'hème est dissocié et éliminé par un agent approprié, par exemple acétonique, la fraction comprenant la globine et d'autres protéines, mise sous forme liquide, est neutralisée, de sorte que la globine est précipitée et, au moins partiellement, séparée des autres constituants protéiques du sang.

Par exemple, le sang humain est prélevé chez un patient et recueilli en présence d'un agent anticoagulant, tel que du citrate de sodium ou de l'héparine, sous agitation. Le prélèvement est aussitôt congelé. Après décongélation, le sang est additionné d'eau distillée pour compléter l'hémolyse puis, de préférence, clarifié par filtration ou centrifugation. La solution de sang clarifié est versée sous agitation dans environ 10 volumes d'acétone acidifiée, à température ambiante. L'acétone contenant l'hème dissout est éliminée par filtration sur toile poreuse. Le précipité de protéines plasmatiques et de globine décolorée est lavé sur la toile par de l'acétone anhydre, et récupéré après essorage. Le précipité est ensuite redissout en solution aqueuse additionnée de NaCl. Une filtration sur membrane poreuse permet d'éliminer les impuretés plasmatiques dénaturées non solubles. Le filtrat est ensuite neutralisé et la globine précipite massivement, en laissant les protéines plasmatiques solubles dans le surnageant. Le précipité de globine neutre, purifiée, est lavé sur une toile de filtration par une solution physiologique. La simplicité du procédé le rend automatisable, ce qui permet une préparation rapide et économique de la globine homologue ou autologue.

De façon surprenante on peut ainsi obtenir des préparations de couleur blanchâtre, concentrées et particulièrement fluides de globine.

L'invention concerne également l'utilisation d'une préparation selon l'invention, pour la réalisation d'une préparation implantable ou injectable dans l'organisme.

L'utilisation selon l'invention permet, notamment d'améliorer la biocompatibilité, la colonisation cellulaire ou l'intégration tissulaire, ou encore la résorption, de l'agent adhésif, ou de comblement ou d'augmentation ou de cicatrisation présent dans la préparation.

Ainsi cette utilisation peut porter, notamment, sur la réalisation d'une préparation adhésive pour :
- cicatrisation, protection ou comblement de plaies cutanées externes ou chirurgicales internes,
- comblement des rides et défauts cutanés,
- comblement des tissus conjonctifs ou sphincters pour des applications en urologie : reflux vésico-urétéral de l'enfant, incontinence d'effort de la femme ; en O.R.L. : correction de volume des cordes vocales,
- bouchon adhésif et hémostatique pour les plaies tissulaires ou les plaies artérielles percutanées,
- moyen de fixation de prothèses ou biomatériaux sur les tissus receveurs, notamment pour la fixation des dispositifs de renfort pariétal et viscéral,
- ou films, gels et membranes pour prévention des adhérences post opératoires, utilisés seuls ou en association avec d'autres dispositifs médicaux.

Enfin l'invention concerne également un procédé de traitement cosmétologique ou thérapeutique, dans lequel on administre à un patient, qui en a besoin, une quantité cosmétologiquement ou thérapeutiquement efficace d'une préparation selon l'invention.

Ce procédé concerne, notamment, les utilisations précitées.

Les exemples qui suivent décrivent la réalisation de l'invention, de manière non limitative, pour améliorer la biocompatibilité des colles et implants actuels, utilisées en médecine et chirurgie.

### Exemples de réalisation de produits selon l'invention.

### Exemple 1: Préparation de globine humaine, insoluble à pH physiologique.

30 ml de sang humain sont prélevés par ponction veineuse d'un patient et recueillis sous agitation avec 3ml de citrate de sodium. Le prélèvement est aussitôt congelé. Après décongélation, le sang est additionné d'un volume égal d'eau distillée stérile pour compléter l'hémolyse, puis centrifugé pendant 30 mn à 10000 t/mn, ou/et clarifié sur des préfiltres et filtré stérilement sur membrane de porosité 0.2 à 0,45 micron. Après rinçage du filtre, on obtient un volume de 74 ml, contenant 52 mg/ml d'hémoglobine qui est conservé à 4° C. Cette solution est lentement versée sous agitation dans 750 ml d'acétone, contenant 7,5ml d'HCl 12 N. La suspension est agitée vigoureusement et laissée au repos pendant 10 à 60mn à température ambiante, sous une hotte chimique ou dans un réacteur étanche. L'acétone contenant l'hème dissout est éliminée par filtration sur toile poreuse. Le précipité de globine décolorée est lavé sur la toile par de l'acétone anhydre, et récupéré après essorage. Un séchage sous vide permet de préparer de la poudre de globine, de couleur blanche, qui est facile à stocker ou transporter, si nécessaire. Le précipité de globine (21,8g), ou la poudre correspondante est ensuite redissout dans 300ml d'eau distillée stérile, puis la solution est additionnée de NaCl 5g/l. Le pH acide (=2,5) est ajusté à une valeur de 5,0 à 5,5 par addition de soude 1 N. La globine reste soluble dans ces conditions et une filtration sur membrane poreuse permet d'éliminer les impuretés plasmatiques dénaturées non solubles. Le filtrat est ensuite ajusté à pH 7,0 et la globine précipite massivement, en laissant les protéines plasmatiques solubles dans le surnageant. Le précipité de globine neutre, purifiée, est lavé sur une toile de filtration par une solution de NaCl 5g/l. Une purification complémentaire peut être réalisée par dissolution acide, filtration sur membrane poreuse stérilisante de 0,2 à 0,45µm et reprécipitation à pH neutre. D'autre part, un traitement alcalin par une concentration finale de soude égale à 0,2N, pendant 1 heure à +20°C, suivi d'une précipitation à pH neutre par addition d'acide chlorhydrique 1 N, permet d'obtenir une garantie d'inactivation complémentaire des agents infectieux ou transmissibles, éventuellement présents dans le sang du patient. En final, la pâte de globine insoluble est mise en suspension dans une solution physiologique de chlorure de sodium 9g/l, éventuellement tamponnée à pH 7. En variante, le précipité de globine peut être lavé en eau distillée, puis soumis à une lyophilisation pour préparer une poudre neutre de globine insoluble. L'addition de hyaluronate de sodium ou d'agents plastifiants tels que la glycérine ou/et le polyéthylène glycol permet de préparer des poudres de globine plus ou moins complexes, en vue de leur mélange avec les biomatériaux adhésifs. En final, la pâte ou la poudre de globine insoluble peut être stérilisée par irradiation beta ou gamma à une dose de 5 à 30kGray.

### Exemple 2: Préparation de globine humaine, modifiée chimiquement, soluble à pH physiologique (exemple non conforme à l'invention).

On réalise le procédé de l'exemple 1 à partir d'une poche de concentré globulaire de 400ml, contrôlée et périmée, obtenue auprès d'un centre de transfusion sanguine. Les volumes de réactifs sont adaptés de manière proportionnelle au volume à traiter. En fin de purification le précipité de globine est redissout à pH acide de 2 à 3. La globine est précipitée dans 10 volumes d'acétone, puis lavée par de l'acétone anhydre et séchée sous vide ou sous courant d'air avec une agitation continue, pour préparer une poudre acide, finement divisée et débarrassée des sels. On pèse 8g de poudre de globine humaine, finement divisée, dans un flacon de 250ml, auquel est ajouté 200ml d'éthanol anhydre, contenant 2ml d'HCl 12N, soit une concentration acide finale de 0,12N. La poudre reste parfaitement insoluble dans ces conditions, gonfle un peu et se disperse bien. Après bouchage hermétique du flacon, une incubation d'une semaine est réalisée à température ambiante sous agitation modérée plusieurs fois par jour.

Le précipité de globine est séparé de l'éthanol acide, à l'aide d'un filtre nylon de porosité 1µm. Le précipité est lavé par trois fois 200ml d'acétone pure, puis séché à l'air pour obtenir 7,4g d'une poudre fine bien divisée. En final, la poudre de globine soluble peut être stérilisée par irradiation beta ou gamma à une dose de 5 à 30kGray.

Cette poudre peu mouillable spontanément, peut être rapidement dissoute dans 200ml d'eau distillée, dans un bécher. On obtient une solution limpide jaune paille, de pH 2,8. La neutralisation du pH de cette solution par addition goutte à goutte de soude 0,5N, sous agitation manuelle et avec contrôle continu du pH permet de vérifier le caractère soluble de la globine à pH neutre entre 6 et 8, puis sa précipitation massive à un pH alcalin voisin de 9,5 à 10.

Il est possible d'observer un début de précipitation vers pH6. Ceci correspond à la présence partielle de globine non modifiée, protégée de la réaction d'estérification au coeur d'un gros agrégat de poudre. Il est à noter que les esters de globine ainsi préparés sont progressivement hydrolysés in vivo et régénèrent spontanément la globine insoluble initiale, qui elle-même sera dégradée. Il s'agit donc d'une modification chimique réversible avec le temps, ou in vivo. Il est à noter aussi que les esters de globine ainsi préparés possèdent une charge électrique positive très marquée. Leur point isoélectrique basique, voisin de 10, provient dé la disparition plus ou moins complète des fonctions carboxyliques. Cette propriété leur confère un caractère adhésif vis-à-vis des tissus chargés négativement. Ces esters de globine possèdent une grande quantité de fonctions amines qui peuvent être facilement réticulées par les réactifs habituels des colles biologiques, dont les fonctions aldéhydes, tels que le glutaraldéhyde, ou les polysaccharides oxydés. En outre, ils peuvent facilement former des réseaux stables grâce à des liaisons électrostatiques fortes, en présence de tous polymères ou biopolymères chargés négativement. Parmi les polymères synthétiques nous pouvons citer par exemple les polymères polyacryliques ou les dérivés anioniques des polyéthylènes glycols. Parmi les polymères naturels, nous pouvons citer le fibrinogène, la fibrine, l'albumine, les hyaluronates, l'héparine, les dérivés biorésorbables de la cellulose tels que la cellulose oxydée, ou tous les autres polysaccharides sulfatés, ou riches en fonctions carboxyliques.

### Exemple 3: Préparation de globine humaine, modifiée chimiquement, insoluble à pH physiologique.

Si l'on remplace l'éthanol utilisé dans l'exemple 2, par un alcool gras, porteur d'une chaîne d'au moins quatre carbones, tel que le butanol, l'hexanol, l'octanol ou même porteur d'une plus longue chaîne, le dérivé de globine estérifiée obtenu n'est plus soluble à pH neutre, mais devient insoluble dans l'eau ou dans une solution aqueuse physiologique. Pour faciliter la réaction d'estérification des fonctions carboxyliques de la globine par un alcool à longue chaîne carbonée, il est préférable d'utiliser des moyens classiques d'activation tels que les carbodiimides ou d'autres agents activateurs connus des fonctions alcools ou carboxyliques.

### Exemple 4: Préparation de globine humaine, modifiée chimiquement, insoluble à pH physiologique.

5 grammes de poudre de globine soluble estérifiée, préparée selon l'exemple 2, sont mis en suspension dans 200ml d'une solution aqueuse d'éthanol à 50%, contenant du glutaraldéhyde à une concentration de 0,25%. Après incubation d'une heure à température ambiante, la suspension de globine réticulée est additionnée de 2 grammes d'hydroborure de sodium, dans une hotte chimique pour l'évacuation de l'hydrogène naissant en excès. Cette réaction rapide assure la réduction des fonctions aldéhydes en excès, et les transforme en fonctions alcool. La suspension de globine ainsi traitée perd la coloration jaune conférée par le glutaraldéhyde. Elle est ensuite lavée en éthanol puis en acétone et enfin séchée sous vide, pour préparer une poudre blanchâtre de globine réticulée insoluble.

### Exemple 5 : Association d'une poudre stérile de globine insoluble avec les colles biologiques commerciales.

La poudre de globine insoluble préparée selon l'un des exemples 1, 3 et 4, peut être utilisée comme additif au composant liquide des colles à usage médical ou chirurgical. Cette poudre ne dilue pas le principe actif (monomères ou polymères solubles) de la colle biologique commerciale. Le mélange des deux composants de ces colles biologiques est possible de la même manière. La globine n'empêche pas la réaction finale de polymérisation ou réticulation de l'ensemble auquel la globine participe, lorsque le catalyseur de polymérisation ou l'agent de réticulation est ajouté. Ce mélange peut être réalisé par un dispositif mélangeur approprié et stérile juste avant l'emploi de la colle, ou peut être intégré dans le procédé de fabrication du composant liquide de la colle biologique. Le réseau de globine inséré à l'intérieur de la colle constitue une voie d'entrée pour les cellules qui vont participer à la reconstruction tissulaire progressive du volume de cette colle et son intégration harmonieuse avec les tissus environnants. Cette addition de globine est facilement réalisée avec le composant soluble des colles implantables actuelles. Par exemple les colles à base de fibrinogène telles que Tisseel®, Beriplast® ; à base d'albumine telles que Bioglue® ; à base de dérivés du polyethylene-glycol telles que Coseal®, Duraseal® ; à base de dérivés acryliques telles que Indermil®, ou Dermabond®.

### Exemple 6 : Association d'une pâte stérile de globine insoluble avec les colles biologiques commerciales.

L'exemple 5 ci-dessus est reproduit en remplaçant la poudre de globine par la pâte aqueuse de globine préparée selon l'un des exemples 1, 3 et 4. La dilution du principe adhésif, apportée par l'eau contenue dans la pâte de globine, est négligeable et largement compensée par la globine additionnelle qui participe au nouveau mélange adhésif, en l'épaississant et en augmentant sa viscosité et sa consistance. Cette eau ne se mélange d'ailleurs pas dans le cas où le composant adhésif est en solution organique non miscible avec l'eau.

### Exemple 7 : Association d'une poudre stérile de globine soluble avec les colles biologiques commerciales.

Les exemples 5 et 6 ci-dessus sont reproduits en utilisant la poudre de globine soluble préparée selon l'exemple 2. Dans ce cas, la globine n'apporte aucune dilution. Après application du nouveau mélange adhésif in vivo, la poudre de globine absorbe progressivement l'eau des tissus environnants, ce qui va constituer la voie d'entrée nouvelle des cellules pour la colonisation, l'intégration tissulaire et la résorption progressive de l'implant.

### Exemple 8 : Association d'une pâte de globine insoluble avec des fibres de cellulose oxydée.

Une pâte de globine insoluble est préparée, de préférence, selon l'un des exemples 1, 3 ou 4. Cette pâte de globine insoluble en suspension, par exemple dans l'eau distillée est mélangée à une pâte de fibres de cellulose oxydée, dans des proportions respectives de 20 et 80%. L'ensemble est lyophilisé et stérilisé par irradiation gamma à une dose de 25 à 30kGray. On obtient une éponge composite de cellulose oxydée et de globine insoluble, pour le traitement des plaies internes ou externes. Les proportions de globine et cellulose oxydée peuvent être modifiées pour optimiser les propriétés de cicatrisation et la vitesse de résorption du biomatériau, la proportion de globine étant inférieure à 50%.

### Exemple 9 : Association d'une pâte de globine insoluble avec un gel de hyaluronate réticulé.

Une pâte de globine insoluble est préparée, de préférence, selon l'un des exemples 1, 3 ou 4. Cette pâte de globine insoluble, de préférence à une concentration de 10%, en suspension dans une solution physiologique, par exemple de 9g/l de NaCl, est répartie en seringue plastique ou verre de 1 ml (Schott ou Becton Dickinson) et stérilisée par irradiation gamma à une dose de 25 à 30kGray. La pâte stérile de globine insoluble est ensuite mélangée à un gel stérile de hyaluronate réticulé et stérile dans toutes proportions possibles, par exemple grâce à un connecteur coupleur stérile, par exemple en polypropylène, de type Luer Lock, fourni par Promepla, Principauté de Monaco, (ref FTLLC-6). Le coupleur peut éventuellement comporter une restriction de diamètre compris entre 0,2 et 2mm. Le hyaluronate réticulé peut être l'un quelconque des produits commerciaux : Restylane® ou Hylaform®, ou Juvederm®, ou Puragen Plus® ou tout autre produit équivalent. Le mélange stérile des deux biopolymères est directement obtenu dans une seringue de préférence de 1ml, en toutes proportions possibles, et peut être injecté pour l'augmentation tissulaire requise. Un tel produit composite permet une colonisation cellulaire de l'implant grâce à la présence même minoritaire de globine insoluble, au sein du gel de hyaluronate réticulé.

D'autres produits ou préparations polymériques de comblement ou augmentation peuvent être associés à la globine naturelle ou modifiée insoluble ou obtenue à partir de globine modifiée pour être au moins partiellement soluble, tels que les acides polylactiques, par exemple selon l'exemple suivant :

### Exemple 10 : Association d'une pâte de globine insoluble avec une suspension de microparticules d'acide polylactique.

Une pâte de globine insoluble est préparée, de préférence, selon l'un des exemples 1, 3 ou 4. Cette pâte de globine insoluble, de préférence à une concentration de 10%, en suspension dans une solution physiologique, par exemple de 9g/l de NaCl, est répartie en seringue plastique ou verre de 1ml (Schott ou Becton Dickinson) et stérilisée par irradiation gamma à une dose de 25 à 30kGray. La pâte stérile de globine insoluble est ensuite mélangée à une suspension stérile de microparticules d'acide polylactique dans toutes proportions possibles, par exemple grâce à un connecteur coupleur stérile, par exemple en polypropylène, de type Luer Lock, fourni par Promepla, Principauté de Monaco, (ref FTLLC-6). Le coupleur peut éventuellement comporter une restriction de diamètre compris entre 0,2 et 2mm. La suspension de microparticules d'acide polylactique peut être par exemple obtenue à partir du produit Sculptra® distribué par la société Dermik, ou toute autre pâte injectable d'acide polylactique destinée au comblement tissulaire. Le mélange stérile des deux biopolymères est directement obtenu dans une seringue de préférence de 1 ml, en toutes proportions possibles, et peut être injecté pour l'augmentation tissulaire requise. Un tel produit composite permet une meilleure colonisation cellulaire de l'implant, plus stable, moins inflammatoire, grâce à la présence même minoritaire de globine insoluble, au sein de la préparation implantée.

### Exemple 11 : Association d'une pâte de globine insoluble avec des particules minérales.

Une pâte de globine insoluble est préparée, de préférence, selon l'exemple 1, 3 ou 4. Cette pâte est mélangée à une suspension de particules minérales constituées de phosphate de calcium ou d'hydroxyapatite, par exemple dans des proportions respectives de 20 et 80%). L'ensemble est séché sous courant d'air stérile ou lyophilisé et stérilisé par irradiation gamma à une dose de 25 à 30kGray. On obtient une poudre composite de particules minérales enrobées (dans le cas du séchage), ou mélangées (dans le cas de la lyophilisation) avec de la globine insoluble, pour le traitement des fractures ou pertes osseuses. Les proportions de globine et particules minérales peuvent être modifiées pour optimiser les propriétés de cicatrisation osseuse et la vitesse de résorption du biomatériau, la proportion de globine étant de préférence inférieure à 50%. Lorsque des prothèses orthopédiques sont revêtues de particules minérales destinées à améliorer leur bio-intégration, l'addition de globine à ce revêtement particulaire est particulièrement indiquée.

Les préparations associant un matériau de globine naturelle ou modifiée et insoluble au pH physiologique et/ou un matériau susceptible d'être obtenu à partir de globine modifiée pour être au moins partiellement soluble à pH physiologique, à des particules minérales, notamment constituées de phosphate de calcium ou d'hydroxyapatite, par exemple selon l'exemple 11, et qui peuvent contenir des proportions respectives de préférence de 10/90 à 90/10, peuvent être utilisées non seulement pour le comblement de tissus mous, par exemple cutanés, notamment des rides, de préférence en associant ledit matériau avec de l'hydroxyapatite, mais aussi pour le comblement et la réparation osseuse, y compris en stomatologie et en chirurgie dentaire, par exemple pour le traitement des parodontites.

### Exemple 12: Applications médicales des biomatériaux et colles biologiques contenant de la globine.

Les biomatériaux et colles biologiques associant de la globine, selon la présente invention, en proportion même minoritaire, peuvent être utilisés dans les applications suivantes non limitatives :
- cicatrisation, protection ou comblement de plaies cutanées externes ou chirurgicales internes.
- comblement des rides et défauts cutanés
- comblement des tissus conjonctifs ou sphincters pour des applications en urologie : reflux vésico-urétéral de l'enfant, incontinence d'effort de la femme ; en O.R.L. : correction de volume des cordes vocales.
- bouchon adhésif et hémostatique pour les plaies tissulaires ou les plaies artérielles percutanées.
- moyen de fixation de prothèses ou biomatériaux sur les tissus receveurs, notamment pour la fixation des dispositifs de renfort pariétal et viscéral.
- films, gels et membranes pour prévention des adhérences post opératoires, utilisés seuls ou en association avec d'autres dispositifs médicaux.
- comblement et cicatrisation de plaies osseuses, revêtement de prothèses orthopédiques.

La présente invention décrit également l'utilisation d'une préparation selon les revendications, pour la réalisation d'une préparation injectable ou implantable, selon l'invention, notamment pour une ou une pluralité des applications susmentionnées.

D'une façon générale, quelle que soit la préparation selon l'invention, les méthodes sont à visée thérapeutique. Des méthodes de comblement de rides peuvent, suivant les personnes concernées, être à visée simplement cosmétique. Dans tous les cas, la méthode selon l'invention comporte l'étape d'implanter localement, dans ou sur le tissu du patient ou de la personne, qui en a besoin, une quantité thérapeutiquement ou cosmétiquement efficace, d'une préparation selon l'invention, notamment pour les applications précitées.

### Bibliographie

ANSON M.L. - MIRSKY A.E. (1930) Protein Coagulation and its reversal. The préparation of insoluble globin, soluble globin and heme. J. Gen. Physiol. 13, 469-476
AUTIO K - KIESVAARA M. - MALKKI Y. - KANKU S. (1984) Chemical and functional properties of blood globin prepared by a new method Journal of Food Science 49, 859-862
BERG J.W. - ORTMEYER D.W. - OTT D.L. - JACKSON R.L. (1953) Comparison of Globin Insulin and NPH Insulin Diabetes, 2, 5, p.365-369
RABINOWITCH I.M. - FOWLER A.F. - BENSLEY E.H. - GORDON A.L. - MOUNTFORD M. (1947) Globin Insulin The Canadian Medical Association J., 56, 6, p.595-605
REINER L. (1939)
   Insulin préparation
   US Patent # 2161198
REINER L. - SEARLE D.S. - LANG E.H. (1939) Insulin préparations with prolonged activity I. Globin Insulin Proc. Soc. Exp. Biol. Med. 40, p.71
ROSSI-FANELLI A. -ANTONINI E. -CAPUTO A. (1958) Studies on the structure of haemoglobin I-Physicochemical properties of human globin Biochem. Biophys. Acta 30, 608-615
SCHULZ F.N. (1898) Der Eiweisskörper des hämoglobins Ztsch. F. physiol. chem. 24, 449-460
TEALE F.W.J. (1959) Cleavage of the haem-protein link by acid methyl-ethyl keton Biochem. Biophys. Acta 35, 543

## Revendications

1. Préparation stérile implantable sur ou dans des tissus organiques, comprenant, sous forme de pâte, gel ou suspension injectables:
- un matériau de globine naturelle ou modifiée et insoluble au pH physiologique biocompatibles et biodégradables dans l'organisme, et
- un agent polymériques de comblement ou augmentation tissulaire, à base d'acide hyaluronique réticulé,
dans laquelle la proportion dudit matériau, par rapport à l'ensemble matériau plus agent polymérique est inférieure ou égale à 50%.

2. Préparation selon la revendication 1, dans laquelle la proportion dudit matériau, Par rapport à l'ensemble matériau plus agent polymérique est inférieure à 50%.

3. Préparation selon la revendication 1, dans laquelle la proportion dudit matériau, par rapport à l'ensemble matériau plus agent polymérique est inférieure ou égale à 25%.

4. Préparation selon la revendication 1, dans laquelle la Proportion dudit matériau, par rapport à l'ensemble matériau plus agent polymérique est inférieure ou égale à 10%.

5. Préparation selon l'une des revendications 1 à 4, destinée notamment à combler ou réparer des défauts tissulaires, cutanés, conjonctifs, vasculaires, viscéraux ou osseux, et de manière générale à protéger ou combler des plaies et participer à leur cicatrisation.

## Patentansprüche

1. Steriles Präparat, das auf oder in organisches Gewebe implantierbar ist und in Form einer injizierbaren Paste, injizierbaren Suspension oder eines injizierbaren Gels umfasst:
- ein natürliches oder modifiziertes und bei physiologischem pH unlösliches Globinmaterial, das im Organismus biokompatibel und bioabbaubar ist, und
- ein Polymer zum Gewebeaufbau oder zur Gewebeaugmentation auf Basis einer vernetzten Hyaluronsäure,
bei der das Verhältnis des Materials in Bezug auf das Gesamtmaterial zuzüglich Polymer kleiner oder gleich 50% ist.

2. Präparat nach Anspruch 1, bei dem der Anteil des Materials in Bezug auf das Gesamtmaterial zuzüglich Polymer kleiner als 50% ist.

3. Präparat nach Anspruch 1, bei dem der Anteil des Materials in Bezug auf das Gesamtmaterial zuzüglich Polymer kleiner als oder gleich 25% ist.

4. Präparat nach Anspruch 1, bei dem der Anteil des Materials in Bezug auf das Gesamtmaterial zuzüglich Polymer kleiner als oder gleich 10% ist.

5. Präparat nach einem der Ansprüche 1 bis 4, insbesondere vorgesehen, um Gewebe-, Haut-, Bindegewebe-, vaskuläre, Viszeral- oder Knochen-Defekte zu füllen oder auszubessern, und in allgemeiner Weise, um Wunden zu schützen oder zu füllen und zu ihrer Heilung beizutragen.

## Claims

1. A sterile preparation, being implantable in or on organic tissues, comprising, under an injectable paste, gel or suspension form,
- a natural globin, or modified globin material being insoluble at physiological pH, biocompatible and biodegradable in the body, and
- a tissue filling or augmenting polymeric agent, based on cross-linked hyaluronic acid,
wherein the ratio of said material, compared to the total of material and polymeric agent, is lower or equal to 50%.

2. A preparation according to claim 1, wherein the ratio of said material, compared to the total of material and polymeric agent, is lower than 50%.

3. A preparation according to claim 1, wherein the ratio of said material, compared to the total of material and polymeric agent, is lower or equal to 25%.

4. A preparation according to claim 1, wherein the ratio of said material, compared to the total of material and polymeric agent, is lower or equal to 10%.

5. A preparation according to one of claims 1 to 4, , especially for filling or repairing tissue, cutaneous, conjunctive, vascular, visceral or bone defects, and in general to protect or fill wounds and to help healing thereof.
